# EUROPÄISCHE PATENTSCHRIFT

(11) **EP 2 594 233 B1**
(45) Veröffentlichungstag und Bekanntmachung des Hinweises auf die Patenterteilung: **27.02.2019**
(21) Anmeldenummer: 12193226.3
(22) Anmeldetag: 19.11.2012
(51) Int. Cl.: A61F 5/01, A61F 13/06

(54) **Orthopädische Bandagenanordnung**
Orthopedic bandage arrangement
Agencement de bandage orthopédique

(30) Priorität: 21.11.2011 DE 202011108091 U
(43) Veröffentlichungstag der Anmeldung: 22.05.2013
(73) Patentinhaber: medi GmbH & Co. KG, 95448 Bayreuth (DE)
(72) Erfinder: Bauer, Patrick, 91275 Auerbach (DE); Kübrich, Wolfgang, 96215 Lichtenfels (DE); Hoffeins, Peter, 95448 Bayreuth (DE); Herold-Herrmann, Alexandra, 95168 Marktleuthen (DE)
(74) Vertreter: Blaumeier, Jörg

(56) Entgegenhaltungen:
- DE-U1-202006 003 245
- US-A1- 2008 294 082
- US-A1- 2009 013 450
- US-A1- 2009 105 623

## Beschreibung

Die Erfindung betrifft eine orthopädische Bandagenanordnung für ein Sprunggelenk, umfassend ein einen sockenartigen Gestrickgrundkörper aufweisendes Gestrickteil und ein einen länglichen Gurtgrundkörper aufweisendes, mit dem Gestrickteil verbindbares Gurtteil.

Derartige orthopädische Bandagenanordnungen sind hinlänglich bekannt und dienen insbesondere der Entlastung und Stabilisierung des Sprunggelenks des Fußes bei chronischen, postoperativen und posttraumatischen Reizzuständen. Dabei weisen konventionelle Bandagenanordnung im getragenen Zustand regelmäßig keine Pronationsbewegungen des Sprunggelenks, d. h. Drehungen des Fußes um seine Längsachse nach außen begünstigende oder unterstützende Wirkung, und somit keinen Schutz gegenüber Supinationsbewegungen des Sprunggelenks, d. h. Drehungen des Fußes um seine Längsachse nach innen, auf.

DE 20 2006 003 245 U1 offenbart eine Sprunggelenkbandage mit einem anatomisch geformten socken- oder strumpfartigen Grundkörper, welcher sich zumindest zwischen einem Unterschenkelabschnitt und einem Fußabschnitt erstreckt und aus einem dehnbaren Textilmaterial ausgebildet ist, wobei die Bandage einen Pronationszügel zur Stabilisierung der Pronationsstellung des Fußes umfasst, wobei der Pronationszügel lateral an der Bandage angeordnet ist und nicht dehnbare Textillage umfasst.

Der Erfindung liegt das Problem zugrunde, eine orthopädische Bandagenanordnung mit einer auf das Sprunggelenk eines Trägers wirkenden Pronationswirkung anzugeben.

Das Problem wird durch eine orthopädische Bandagenanordnung gemäß Anspruch 1 gelöst.

Die erfindungsgemäße orthopädische Bandagenanordnung bietet dem Träger zusätzlich zu den üblichen Entlastungs- bzw. Stabilisierungsfunktionen des Sprunggelenks auch einen durch die Pronationswirkung des von dem Gurtgrundkörper abragenden Gurtabschnitts bedingten Supinationsschutz. Mithin unterstützt der Gurtabschnitt im verbundenen Zustand des Gurtteils mit dem Gestrickteil, d. h. bei ordnungsgemäßer Befestigung des Gurtteils an dem Gestrickteil, welche insbesondere über eine sogenannte Achterzügelführung erfolgt, die Pronationsmöglichkeit des Fußes und mindert die Supinationsmöglichkeit des Fußes. Derart sind auf Supinationsbewegungen des Fußes zurückzuführende Belastungen des Sprunggelenks reduziert.

Durch den von diesem winklig, insbesondere in einem Winkel zwischen 10° und 90°, insbesondere zwischen 20° und 45°, besonders bevorzugt von ca. 25°, im Sinne einer Abzweigung abragenden Gurtabschnitt weist bzw. zeigt dieser im getragenen Zustand der Bandagenanordnung, wenn also das Gurtteil ordnungsgemäß mit dem Gestrickteil verbunden bzw. an diesem befestigt ist, im Wesentlichen in vertikaler, d. h. in proximaler Richtung. Insgesamt lässt sich die Form des Gurtteils durch den von diesem abragenden Gurtabschnitt als Y-Form beschreiben.

Zur Verbindung des Gurtteils mit dem Gestrickteil ist es zweckmäßig vorgesehen, dass an den freien Enden des Gurtgrundkörpers sowie an dem freien Ende des Gurtabschnitts jeweils wenigstens ein ein Verbindungselement aufweisender Befestigungsabschnitt zur lösbaren Verbindung des Gurtgrundkörpers mit dem Gestrickgrundkörper vorgesehen ist. Demnach ist das Gurtteil über mehrere Anbindungspunkte, d. h. Anbindungspunkte an wenigstens einem freien Ende des Gurtgrundkörpers und dem freien Ende des Gurtabschnitts, lösbar an dem Gestrickgrundkörper festlegbar. Mithin liegen das Gestrickteil und das Gurtteil als separate, die erfindungsgemäße Bandagenanordnung bildende Komponenten vor. Dies hat insbesondere Vorteile bezüglich der praktischen Handhabung der Bandagenanordnung, so kann beispielsweise das Gestrickteil getrennt von dem Gurtteil gereinigt werden und umgekehrt.

Gleichermaßen ist es denkbar, die Abmessungen des Gurtteils, d. h. insbesondere die Länge des Gurtgrundkörpers respektive des Gurtabschnitts durch einen Bediener für einen Träger der Bandagenanordnung individuell festzulegen, d. h. zu konfektionieren bzw. anzupassen. Die Abmessungen des Gurtteils, d. h. die Länge des Gurtgrundkörpers respektive des Gurtabschnitts, können sonach auf einfache Weise individuell auf einen Träger der Bandagenanordnung angepasst werden. Da es sich bei dem Gurtteil um ein Textil handelt, kann der Gurtgrundkörper respektive der von diesem abragende Gurtabschnitt einfach mit einer Schere oder dergleichen auf ein gewünschtes Maß gekürzt werden.

Dabei ist es möglich, dass wenigstens ein gurtgrundkörperseitiger Befestigungsabschnitt lösbar mit dem Gurtgrundkörper und/oder der gurtabschnittsseitige Befestigungsabschnitt lösbar mit dem Gurtabschnitt verbunden ist. Mithin können sämtliche oder ein Teil der dem Gurtteil, d. h. dem Gurtgrundkörper bzw. Gurtabschnitt zugeordneten Befestigungsabschnitte von dem Gurtgrundkörper bzw. dem Gurtabschnitt gelöst und mit diesen wieder verbunden werden. Grundsätzlich können die Befestigungsabschnitte gleichartig ausgebildet sein, so dass ein entsprechender Befestigungsabschnitt gleichermaßen an einem freien Ende des Gurtgrundkörpers aber auch an dem freien Ende des Gurtabschnitts anordbar ist.

Denkbar ist es auch, die lösbaren Befestigungsabschnitte mit einem Kodierungsmittel oder Identifikationsmittel zu versehen, welches korrespondierenden gurtteilseitigen bzw. gurtgrundkörperseitigen Kodierungsmitteln bzw. Identifikationsmitteln eindeutig zuordbar ist, so dass ein entsprechendes befestigungsabschnittsseitig vorgesehenes Kodierungs- bzw. Identifikationsmittel eindeutig einem bestimmten freien Ende des Gurtgrundkörpers oder des Gurtabschnitts zuordbar ist. Ein entsprechendes Kodierungsmittel bzw. Identifikationsmittel kann beispielsweise ein eine Zeichenfolge, wie eine Nummerierung oder eine Beschriftung, darstellendes Gestaltungselement und/oder ein farbliches Gestaltungselement sein, anhand welchem eindeutig erkennbar ist, welche Befestigungsabschnitte an welchem freien Ende des Gurtteils, d. h. des Gurtgrundkörpers bzw. des Gurtabschnitts, anzuordnen ist.

Bevorzugt ist die Verbindung des wenigstens einen mit dem Gurtgrundkörper zu verbindenden Befestigungsabschnitts und/oder des mit dem Gurtabschnitt zu verbindenden Befestigungsabschnitts über ein befestigungsabschnittsseitig vorgesehenes Verbindungselement und ein dazu korrespondierendes gurtgrundkörperseitiges und/oder gurtsabschnittsseitiges Verbindungselement gebildet. Die korrespondierenden Verbindungselemente erlauben eine, insbesondere per Hand, lösbare Verbindung der befestigungsabschnittsseitigen Verbindungselemente mit den gurtgrundkörperseitigen bzw. gurtabschnittsseitigen Verbindungselementen. Im verbundenen Zustand entsprechender Verbindungselemente liegt eine, insbesondere im Hinblick auf beim Tragen der Bandagenanordnung entstehende bzw. herrschende Kräfte, stabile Verbindung vor.

Der Befestigungsabschnitt kann eine von diesem abragende, ein Verbindungselement aufweisende Zunge aufweisen, so dass der Befestigungsabschnitt mit einem korrespondierenden Verbindungselement auf der Ober- und Unterseite des Gurtgrundkörpers und/oder des Gurtabschnitts verbindbar ist. Grundsätzlich ist der Befestigungsabschnitt vorteilhaft einseitig mit einem entsprechenden Verbindungselement versehen. Durch die zusätzliche Anbringung der ebenfalls ein, insbesondere gleichartiges, Verbindungselement aufweisenden Zunge weist der Befestigungsabschnitt sonach einen Bereich sich gegenüberliegender Verbindungselemente auf, welcher es ermöglicht, den Befestigungsabschnitt mit dem Gurtgrundkörper respektive dem Gurtabschnitt ober- und unterseitig zu verbinden. Hierbei umgreifen die befestigungsabschnittsseitigen Verbindungselemente die freien Enden des Gurtgrundkörpers respektive des Gurtabschnitts und verbinden sich mit den daran vorgesehenen Verbindungselementen. Es ergibt sich eine besonders stabile Verbindung. Mithin nimmt der Befestigungsabschnitt die Gestalt eines "Y" an, wobei die beiden von der sich vertikal erstreckenden Linie winklig abragenden Schenkel an ihren sich gegenüberliegenden Seiten mit einem Verbindungselement versehen sind.

Zur Anordnung bzw. Befestigung des Gurtteils an dem Gestrickteil ist zweckmäßig an dem Gestrickgrundkörper ein, insbesondere flügelförmiger, ein Verbindungselement aufweisender Befestigungsabschnitt angebracht, welcher mit einem mit einem freien Ende des Gurtgrundkörpers verbundenen, ein korrespondierendes Verbindungselement aufweisenden Befestigungsabschnitt verbindbar ist. Die flügelförmige Ausbildung des gestrickgrundkörperseitigen Befestigungsabschnitts ist dadurch bedingt, dass der Befestigungsabschnitt bezüglich seiner Längsachse mittig durch eine sich vom Fersenbereich in proximaler Richtung erstreckenden Naht des Gestrickgrundkörpers am Gestrickgrundkörper angebracht ist, so dass die der Naht benachbarten Seitenbereiche bzw. "Flügel" des Befestigungsabschnitts nicht mit dem Gestrickgrundkörper verbunden sind, sondern von diesem abragen. Die Seitenbereiche bzw. "Flügel" können gegebenenfalls entfernt bzw. abgeschnitten werden, sofern eine Anbringung des Gurtteils an dem Gestrickteil nicht mehr gewünscht bzw. erforderlich ist, in welchem Fall das Gestrickteil selbstverständlich weiterhin als Bandage verwendbar ist.

Der gestrickgrundkörperseitige Befestigungsabschnitt ist vorteilhaft am Außenumfang des Gestrickgrundkörpers angeordnet. Dabei setzt der Befestigungsabschnitt mit einem oberen Rand, insbesondere unter Beibehaltung eines Freiraums, d. h. insbesondere nicht bündig, an einem Abschlussrand des Gestrickgrundkörpers an. Grundsätzlich sind selbstverständlich auch andere Anbringungspunkte des gestrickgrundkörperseitigen Befestigungsabschnitts am Gestrickgrundkörper denkbar. In Ausnahmefällen kann der obere Rand des gestrickgrundkörperseitigen Befestigungsabschnitts auch bündig mit einem Abschlussrand des Gestrickgrundkörpers abschließen.

Bevorzugt ist der gestrickgrundkörperseitige Befestigungsabschnitt aus einem undehnbaren Material gebildet. Derart ist dieser widerstandsfähiger gegen beim Tragen der Bandagenanordnung auftretende Kräfte, d. h. insbesondere Scher- und/oder Zugkräfte.

Der Gurtgrundkörper besteht aus einem dehnbaren und einem mit diesem verbundenen undehnbaren Abschnitt, wobei der Gurtabschnitt von dem undehnbaren Abschnitt abragt. Mithin weist das Gurtteil durch die zweiteilige Ausführung des Gurtgrundkörpers aus dem dehnbaren und dem undehnbaren Abschnitt, welche insbesondere über eine Naht miteinander verbunden sind, eine Dehnbarkeit bezogen auf den getragenen Zustand der Bandageanordnung nach Umschlingung des Fußes an dem Gurtgrundkörper festzulegenden Bereich des Gurtgrundkörpers auf. Derart ist der zuletzt im Rahmen der Verbindung des Gurtteils mit dem Gestrickteil festzulegende Bereich des Gurtgrundkörpers mit einer gewissen Dehnbarkeit versehen. Dehnbarkeit ist im erfindungsgemäßen Sinne so zu verstehen, dass der jeweilige Abschnitt per Hand in Längsrichtung um ein gewisses Maß dehnbar bzw. verlängerbar ist. Die grundsätzliche Dehnbarkeit eines Materials bei Anlegen einer genügend hohen Kraft ist hierunter nicht zu verstehen.

Der Gurtabschnitt ist in vorteilhafter Weiterbildung der Erfindung einstückig mit dem undehnbaren Abschnitt ausgebildet. Mithin ragt der Gurtabschnitt winklig von dem undehnbaren Abschnitt des Gurtgrundkörpers ab und besteht aus demselben undehnbaren Material wie dieser. Durch die einstückige Ausbildung des Gurtabschnitts mit dem undehnbaren Abschnitt des Gurtgrundkörpers ist der Gurtabschnitt ebenso undehnbar, wodurch seine Pronationswirkung im getragenen Zustand der Bandagenanordnung verbessert ist.

Es ist möglich, dass der undehnbare Abschnitt ober- und unterseitig komplett mit einem Verbindungselement versehen ist. Hierdurch ergibt sich der Vorteil, dass der undehnbare Abschnitt, d. h. gegebenenfalls auch der von diesem abragende Gurtabschnitt, eine Vielzahl an möglichen Anbindungspunkten für einen ein korrespondierendes Verbindungselement aufweisenden Befestigungsabschnitt des Gurtteils oder des Gestrickteils bietet. Insbesondere kann der undehnbare Abschnitt auch beidseitig, d. h. ober- und unterseitig mit einem korrespondierenden Verbindungselement verbunden werden, wodurch sich eine besonders stabile Verbindung des undehnbaren Abschnitts mit einem daran festzulegenden Bereich des dehnbaren Abschnitts des Gurtgrundkörpers oder einem daran festzulegenden Bereich des Gestrickteils ergibt.

Ein Verbindungselement weist insbesondere mehrere Klettverschlusselemente, insbesondere Flauschelemente oder Hakenelemente auf. Ein Klettverschluss bietet die Möglichkeit der Herstellung lösbarer Verbindungen zwischen zwei mit korrespondierenden Verbindungselementen, d. h. entweder Flausch- oder Hakenelementen, worunter selbstverständlich Bereiche mit entsprechenden Flausch- oder Hakenelementen zu verstehen sind, versehenen Teilen.

Der Gurtgrundkörper kann wenigstens eine, sich insbesondere in Längsrichtung des Gurtgrundkörpers erstreckende, längliche Durchbrechung aufweisen. Die im Wesentlichen schlitzartige Durchbrechung verhindert Faltenbildungen des Gurtteils beim Anlegen des Gurtgrundkörpers bzw. des Gurtteils an dem Gestrickgrundkörper bzw. dem Gestrickteil respektive im getragenen Zustand der Bandagenanordnung und erhöht sonach den Tragekomfort der Bandagenanordnung. Mithin ist eine Faltenbildung des Gurtteils auf dem Fußrücken des Trägers verhindert. Die wenigstens eine Durchbrechung ist bevorzugt in dem undehnbaren Abschnitt des Gurtgrundkörpers ausgebildet.

Ferner ist der Gestrickgrundkörper beispielsweise aus einem elastischen Faden gestrickt. Es handelt sich dabei insbesondere um ein Flachgestrick. Die Verwendung eines elastischen Fadens verleiht dem Gestrickgrundkörper eine gewisse Elastizität bzw. Dehnbarkeit, so dass dieser an unterschiedliche Abmessungen eines Trägerfußes anpassbar ist. Der elastische Faden oder ein mit dem elastischen Faden zur Bildung des Gestrickgrundkörpers verstrickter Faden weist vorteilhaft Hafteigenschaften gegenüber der Haut eines Trägers auf, so dass die Möglichkeit eines unerwünschten, insbesondere proximalen und/oder distalen und/oder um den Unterschenkel umfangsmäßigen, Verrutschens des Gestrickteils verhindert ist. Der Faden kann beispielsweise aus Nylon bzw. Polyamid (PA) bestehen.

Der Gestrickgrundkörper kann wenigstens eine, sich im getragenen Zustand, insbesondere über das Sprunggelenk erstreckende, orthopädische Pelotte aufweisen. Die Pelotte erhöht die orthopädisch wirksame Funktion des Gestrickgrundkörpers bzw. des Gestrickteils und somit der Bandagenanordnung insgesamt. Die Pelotte kann zu unterschiedlichen orthopädischen Zwecken in unterschiedlichen Positionen in oder an dem Gestrickgrundkörper angeordnet sein. Dabei ist es möglich, dass die Pelotte in den Gestrickgrundkörper integriert ist, so dass sich eine lagefeste und unverlierbare Verbindung der Pelotte mit dem Gestrickgrundkörper ergibt.

Weitere Vorteile, Merkmale und Einzelheiten der Erfindung ergeben sich aus den im Folgenden beschriebenen Ausführungsbeispielen sowie anhand der Zeichnungen. Dabei zeigen:
- Fig. 1: eine Prinzipdarstellung einer Bandagenanordnung in einer beispielhaften Ausführungsform;
- Fig. 2: eine Prinzipdarstellung eines Gestrickteils in einer beispielhaften Ausführungsform;
- Fig. 3: eine Ansicht des Fersenbereichs des Gestrickteils gemäß Fig. 2;
- Fig. 4: eine Prinzipdarstellung der Oberseite eines Gurtteils in einer beispielhaften Ausführungsform;
- Fig. 5: eine Prinzipdarstellung der Unterseite eines Gurtteils in einer beispielhaften Ausführungsform;
- Fig. 6: eine Prinzipdarstellung eines gurtabschnittsseitig anzuordnenden Befestigungsabschnitts in einer beispielhaften Ausführungsform; und
- Fig. 7: eine Prinzipdarstellung der Verbindung eines Befestigungsabschnitts mit dem freien Ende eines von dem Gurtgrundkörper des Gurtteils abragenden Gurtabschnitts.

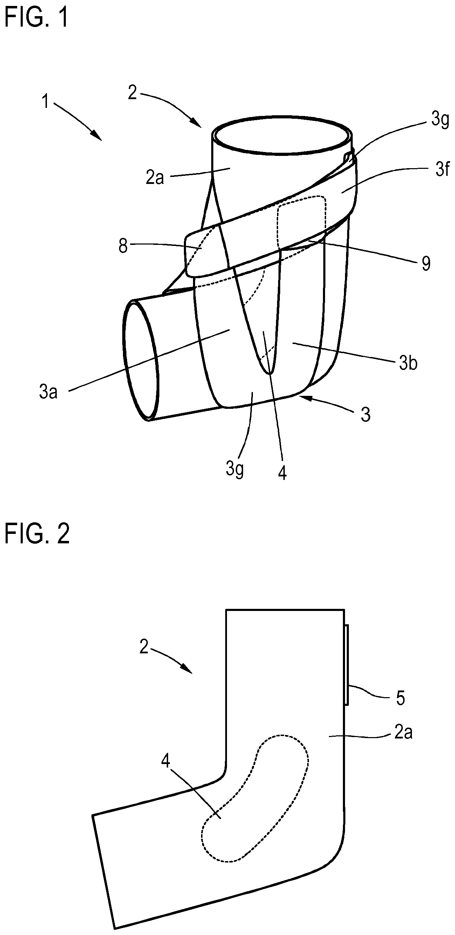

Fig. 1 zeigt eine Prinzipdarstellung einer orthopädischen Bandagenanordnung 1 für ein Sprunggelenk eines Trägers in einer beispielhaften Ausführungsform der Erfindung. Es handelt sich dabei um eine Darstellung der Bandagenanordnung 1 im getragenen Zustand. Der Bandagenanordnung 1 ist ein einen sockenartigen Gestrickgrundkörper 2a aufweisendes Gestrickteil 2 und ein einen länglichen Gurtgrundkörper 3a aufweisendes, mit dem Gestrickteil 2 verbindbares Gurtteil 3 zugehörig. Wie aus Fig. 1 ersichtlich, ragt von dem Gurtgrundkörper 3a ein mit dem Gestrickgrundkörper 2a verbindbarer Gurtabschnitt 3b ab. Im in Fig. 1 gezeigten getragenen Zustand der Bandagenanordnung 1, wobei das Gurtteil 3 mit dem Gestrickteil 2 verbunden ist, zeigt der Gurtabschnitt 3b in proximaler Richtung, d. h. im Wesentlichen in vertikaler Richtung nach oben und übt eine Pronationswirkung auf den Fuß bzw. das Sprunggelenk des Trägers aus, wodurch die Möglichkeit von Supinationsbewegungen des Fußes im Sinne eines Supinationsschutzes des Sprunggelenks gehindert bzw. erschwert wird. Ersichtlich ist das Gurtteil 3 in einer so genannten Achterzügelführung um das Gestrickteil 2 gewunden. Überdeckte Abschnitte bzw. Bereiche des Gurtteils 3 sowie der in Fig. 2 näher gezeigten Pelotte 4 sind gestrichelt dargestellt.

Fig. 2 zeigt eine Prinzipdarstellung eines Gestrickteils 2 in einer beispielhaften Ausführungsform. Das Gestrickteil 2 respektive der Gestrickgrundkörper 2a liegt als aus einem elastischen Faden, wie z. B. einem Polyamidfaden, gebildetes Flachgestrick vor. Der den Gestrickgrundkörper 2a bildende Faden ist insbesondere so gewählt, dass dieser, und somit der Gestrickgrundkörper 2a insgesamt, eine lagefeste bzw. verrutschsichere Anordnung des Gestrickteils 2 an dem Bein- bzw. Fußabschnitt des Trägers ermöglicht, weshalb der Faden vorteilhaft ein gewisses Haftungsvermögen gegenüber der Haut eines Trägers zeigt. In den Gestrickgrundkörper 2a ist eine orthopädisch wirksame Pelotte 4 eingearbeitet, welche sich im getragenen Zustand der Bandagenanordnung 1 über das Sprunggelenk des Trägers erstreckt.

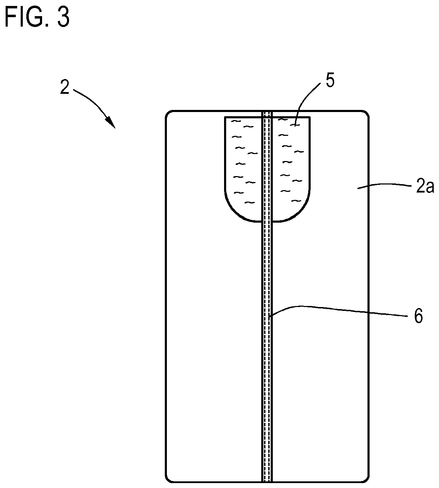

Wie aus Fig. 3, die eine Ansicht des Fersenbereichs des Gestrickteils 2 gemäß Fig. 2 zeigt, zu erkennen ist, ist an dem Gestrickgrundkörper 2a ein flügelförmiger Befestigungsabschnitt 5 angebracht. Der Befestigungsabschnitt 5 ist sonach am Außenumfang des Gestrickgrundkörpers 2a angeordnet. Ersichtlich schließt der obere Rand des Befestigungsabschnitts 5 nicht bündig, sondern unter Beibehaltung eines bestimmten Abstands von beispielsweise ca. 5 mm an dem oberen Abschlussrand bzw. Saum des Gestrickgrundkörpers 2a an. Die Flügelform des Befestigungsabschnitts 5 ergibt sich dadurch, dass der Befestigungsabschnitt 5 über eine sich in distaler Richtung erstreckende, diesen mittig durchlaufende Naht 6 am Gestrickgrundkörper 2a angebracht ist. Die an die Naht 6 angrenzenden Randbereiche des Befestigungsabschnitts 5 sind nicht mit dem Gestrickgrundkörper 2a vernäht respektive nicht an diesem festgelegt.

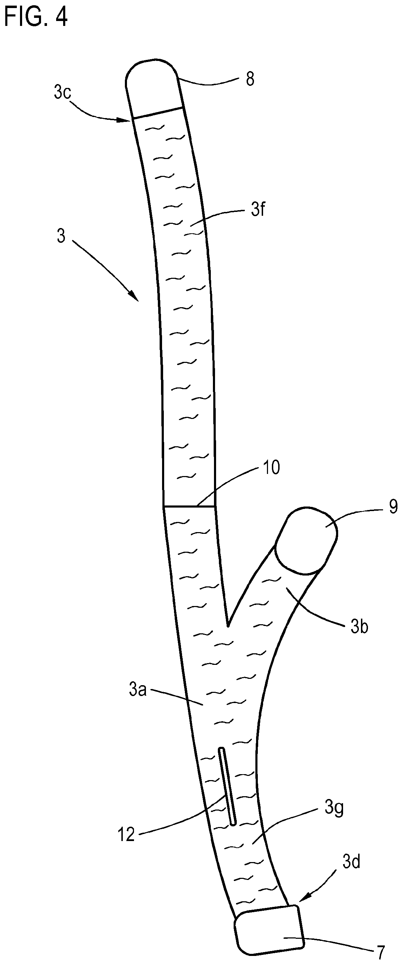

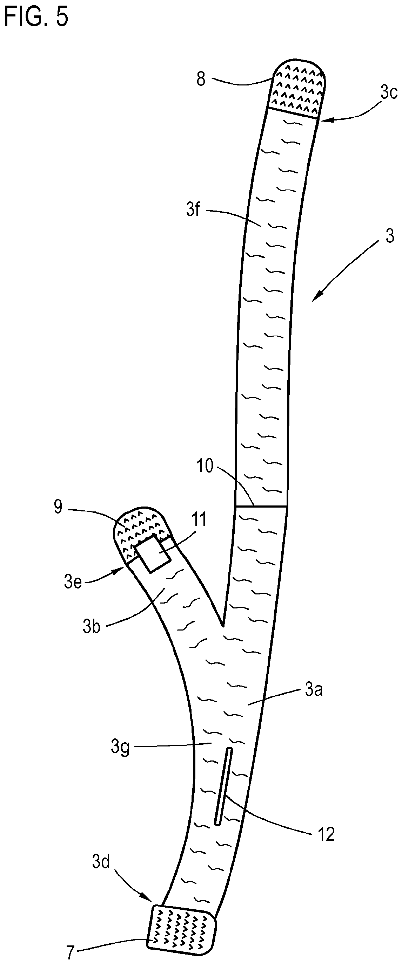

Der aus einem undehnbaren Textilmaterial gebildete Befestigungsabschnitt 5 ist mit seiner von dem Gestrickgrundkörper 2a abragenden Fläche vollständig mit einem Verbindungselement aufweisend mehrere als Flauschelemente gebildete Klettverschlusselemente versehen. Das Verbindungselement dient der lösbaren Anordnung eines mit dem freien Ende 3d des Gurtgrundkörpers 3a verbundenen, ein korrespondierendes Verbindungselement aufweisend mehrere als Hakenelemente ausgebildete Klettverschlusselemente aufweisenden gurtgrundkörperseitigen Befestigungsabschnitts 7 (vgl. Fig. 4, 5) und somit der lösbaren Anbringung des freien Endes 3d des Gurtgrundkörpers 3a an dem Befestigungsabschnitt 5 des Gestrickgrundkörpers 2a. Sofern eine Verbindung des Gestrickteils 2 mit dem Gurtteil 3 nicht mehr gewünscht ist, können die "Flügel" des gestrickgrundkörperseitigen Befestigungsabschnitts 5 etwa durch Abschneiden entfernt werden.

Fig. 4 zeigt eine Prinzipdarstellung der Oberseite eines Gurtteils 3 in einer beispielhaften Ausführungsform. Fig. 5 zeigt eine Prinzipdarstellung der Unterseite eines Gurtteils 3 in einer beispielhaften Ausführungsform. Ersichtlich weist das Gurtteil 3 eine Art Y-Form auf, was sich durch den von dem länglichen Gurtgrundkörper 3a winklig, d. h. in einem Winkel α von ca. 25°, abragenden Gurtabschnitt 3b ergibt. An den freien Enden 3c, 3d des Gurtgrundkörpers 3a sowie an dem freien Ende 3e des Gurtabschnitts 3b sind jeweils Befestigungsabschnitte 7, 8, 9 zur lösbaren Verbindung des Gurtteils 3 mit dem Gestrickteil 2 angeordnet. Die Befestigungsabschnitte 7, 8, 9 weisen in flächiger Anordnung jeweils mehrere als Hakenelemente ausgebildete Klettverschlusselemente auf.

Das Gurtteil 3 respektive der Gurtgrundkörper 3a ist zweiteilig aus einem aus einem dehnbaren Textilmaterial gebildeten dehnbaren Abschnitt 3f und einem aus einem undehnbaren Textilmaterial gebildeten undehnbaren Abschnitt 3g gebildet, welche über eine Naht 10 miteinander verbunden sind. Der Gurtabschnitt 3b ist einteilig mit dem undehnbaren Abschnitt 3g ausgebildet, d. h. der Gurtabschnitt 3b besteht aus dem gleichen Material wie der undehnbare Abschnitt 3g, und ragt von diesem ab. Der undehnbare Abschnitt 3g ist vollständig ober- und unterseitig mit einem Verbindungselement in Form von als Flauschelemente ausgebildeten Kletteverschlusselementen versehen.

Die an den freien Enden 3c, 3d des Gurtgrundkörpers 3a angeordneten Befestigungsabschnitte 7, 8 sind beispielsweise durch Vernähen fest mit dem Gurtgrundkörper 3a verbunden. Dahingegen ist der an dem freien Ende 3e des Gurtabschnitts 3b angeordnete Befestigungsabschnitt 9 über eine Klettverschlussverbindung an dem freien Ende 3e des Gurtabschnitts 3b angebracht und lässt sich somit von dem freien Ende 3e Gurtabschnitts 3b lösen. Dies hat den Vorteil, dass die Längenabmessungen des Gurtabschnitts 3b individuell auf einen Träger angepasst werden können, d. h. der Gurtabschnitt 3b bedarfsgerecht auf ein trägerspezifisches Maß konfektionierbar bzw. kürzbar ist. Hierfür wird gegebenenfalls der mit dem freien Ende 3e des Gurtabschnitts 3b über eine Klettverschlussverbindung verbundene Befestigungsabschnitt 9 entfernt, der Gurtabschnitt 3b auf ein entsprechendes Maß gekürzt und der Befestigungsabschnitt 9 wieder an dem freien Ende 3e des Gurtabschnitts 3b angebracht.

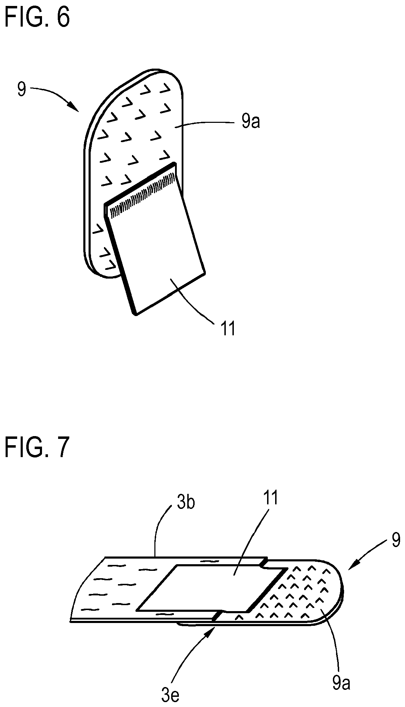

Fig. 6 zeigt eine Prinzipdarstellung eines gurtabschnittsseitig anzuordnenden Befestigungsabschnitts 9 in einer beispielhaften Ausführungsform. Fig. 7 zeigt eine Prinzipdarstellung der Verbindung eines gurtabschnittsseitig anzuordnenden Befestigungsabschnitts 9 mit dem freien Ende 3e des von dem Gurtgrundkörper 3a des Gurtteils 3 abragenden Gurtabschnitt 3b. Ersichtlich ist der gurtabschnittsseitig anzuordnende Befestigungsabschnitt 9 zweiteilig ausgeführt, d. h. erweist ein einseitig komplett mit einem Verbindungselement aufweisend mehrere als Hakenelemente ausgebildete Klettverschlusselemente versehenes Grundteil 9a auf, an welchem eine ebenfalls einseitig mit einem Verbindungselement aufweisend mehrere als Hakenelemente ausgebildete Klettverschlusselemente versehene, von dem Grundteil 9a abragende Zunge 11 befestigt ist. Mithin befinden sich die grundteilseitigen sowie die zungenseitigen Verbindungselemente in Form von Hakenelementen in gegenüber liegender Anordnung, so dass diese sich mit den auf der Ober- bzw. Unterseite des Gurtabschnitts 3b befindlichen Verbindungselementen in Form von Flauschelementen lösbar verbinden lassen.

Die Anbringung der Zunge 11 an das Grundteil 9a ist derart, dass das freie Ende der Zunge 11 über das freie Ende des Grundteils 9a übersteht. Die Länge und der Anbindungspunkt der Zunge 11 sind somit derart gewählt, dass die Zunge 11 das Grundteil 9a in Längsrichtung mit einem gewissen Übermaß überragt. Wie aus Fig. 7 ersichtlich ist, übergreift der Befestigungsabschnitt 9 das freie Ende 3e des Gurtabschnitts 3b ober- und unterseitig, was zu einer, besonders stabilen Befestigung des Befestigungsabschnitts 9 an dem Gurtabschnitt 3b führt.

Wie sich aus den Fig. 3, 4 ferner ergibt, ist an dem Gurtteil 3 eine längliche, sich in Längsrichtung des Gurtteils 3 bzw. des Gurtgrundkörpers 3a erstreckende schlitzartige Durchbrechung 12 vorgesehen, welche der Faltenbildung des Gurtteils 3 im getragenen Zustand der Bandagenanordnung 1 entgegen wirkt. Die Durchbrechung 12 befindet sich im undehnbaren Abschnitt 3g des Gurtgrundkörpers 3a, da sich dieser im getragenen Zustand der Bandagenanordnung 1 im Wesentlichen über den Fußrücken des Trägers erstreckt.

## Patentansprüche

1. Orthopädische Bandagenanordnung (1) für ein Sprunggelenk, umfassend ein einen sockenartigen Gestrickgrundkörper (2a) aufweisendes Gestrickteil (2) und ein einen länglichen Gurtgrundkörper (3a) aufweisendes, mit dem Gestrickteil (2) verbindbares Gurtteil (3), wobei von dem Gurtgrundkörper (3a) wenigstens ein Gurtabschnitt (3b) abragt, welcher im getragenen Zustand der Bandagenanordnung (1), in dem das Gurtteil (3) mit dem Gestrickteil (2) verbunden ist, sich an der Außenseite des Fußes erstreckt
**dadurch gekennzeichnet,**
**dass** der Gurtabschnitt (3b) im getragenen Zustand der Bandagenanordnung sich über das Sprunggelenk erstreckt und mit seinem freien Ende (3e) mit dem Gestrickgrundkörper (2a) und/oder dem Gurtgrundkörper (3a) verbunden ist und dadurch eine Pronationswirkung auf das Sprunggelenk und einen Supinationsschutz auf das Sprunggelenk des Trägers aufweist.

2. Orthopädische Bandagenanordnung nach Anspruch 1, **dadurch gekennzeichnet, dass** der Gurtabschnitt (3b) von dem Gurtgrundkörper (3a) in einem Winkel (a) zwischen 10 und 90°, insbesondere zwischen 20 und 45°, besonders bevorzugt von 25°, abragt.

3. Orthopädische Bandagenanordnung nach Anspruch 1 oder 2, **dadurch gekennzeichnet, dass** an den freien Enden (3c, 3d) des Gurtgrundkörpers (3a) sowie an dem freien Ende (3e) des Gurtabschnitts (3b) jeweils wenigstens ein ein Verbindungselement aufweisender Befestigungsabschnitt (7, 9) zur lösbaren Verbindung des Gurtteils (3) mit dem Gestrickteil (2) vorgesehen ist, wobei vorzugsweise wenigstens ein gurtgrundkörperseitiger Befestigungsabschnitt (7, 8) lösbar mit dem Gurtgrundkörper (3a) und/oder vorzugsweise der gurtabschnittsseitige Befestigungsabschnitt (9) lösbar mit dem Gurtabschnitt (3b) verbunden ist.

4. Orthopädische Bandagenanordnung nach Anspruch 3, **dadurch gekennzeichnet, dass** die Verbindung des wenigstens einen mit dem Gurtgrundkörper (3a) zu verbindenden Befestigungsabschnitts (8) und/oder des mit dem Gurtabschnitt (3b) zu verbindenden Befestigungsabschnitts (9) über ein befestigungsabschnittsseitig vorgesehenes Verbindungselement und ein dazu korrespondierendes gurtgrundkörperseitiges und/oder gurtabschnittsseitiges Verbindungselement gebildet ist.

5. Orthopädische Bandagenanordnung nach Anspruch 3 oder 4, **dadurch gekennzeichnet, dass** der Befestigungsabschnitt (9) eine von diesem abragende, ein Verbindungselement aufweisende Zunge (11) aufweist, so dass der Befestigungsabschnitt (9) mit einem korrespondierenden Verbindungselement auf der Ober- und Unterseite des Gurtgrundkörpers (3a) und/oder des Gurtabschnitts (3b) verbindbar ist.

6. Orthopädische Bandagenanordnung nach einem der vorangehenden Ansprüche, **dadurch gekennzeichnet, dass** an dem Gestrickgrundkörper (2a) ein, insbesondere flügelförmiger, ein Verbindungselement aufweisender Befestigungsabschnitt (5) angebracht ist, welcher mit einem mit einem freien Ende (3d) des Gurtgrundkörpers (3a) verbundenen, ein korrespondierendes Verbindungselement aufweisenden Befestigungsabschnitt (7) verbindbar ist, wobei vorzugsweise der gestrickgrundkörperseitige Befestigungsabschnitt (5) am Außenumfang des Gestrickgrundkörpers (2a) angeordnet ist, und/oder vorzugsweise der gestrickgrundkörperseitige Befestigungsabschnitt (5) aus einem undehnbaren Material gebildet ist.

7. Orthopädische Bandagenanordnung nach einem der vorangehenden Ansprüche, **dadurch gekennzeichnet, dass** der Gurtgrundkörper (3a) aus einem dehnbaren Abschnitt (3f) und einem mit diesem verbundenen undehnbaren Abschnitt (3g) besteht, wobei der Gurtabschnitt (3b) von dem undehnbaren Abschnitt (3g) abragt.

8. Orthopädische Bandagenanordnung nach Anspruch 7, **dadurch gekennzeichnet, dass** der Gurtabschnitt (3b) einstückig mit dem undehnbaren Abschnitt (3g) ausgebildet ist, und/oder der undehnbare Abschnitt (3g) ober- und unterseitig komplett mit einem Verbindungselement versehen ist.

9. Orthopädische Bandagenanordnung nach einem der Ansprüche 3 bis 8, **dadurch gekennzeichnet, dass** ein Verbindungselement mehrere Klettverschlusselemente, insbesondere Flauschelemente oder Hakenelemente, aufweist.

10. Orthopädische Bandagenanordnung nach einem der vorangehenden Ansprüche, **dadurch gekennzeichnet, dass** der Gurtgrundkörper (3a) wenigstens eine, sich insbesondere in Längsrichtung des Gurtgrundkörpers (3a) erstreckende, längliche Durchbrechung (12) aufweist.

11. Orthopädische Bandagenanordnung nach einem der Ansprüche 7 bis 9 und Anspruch 10, **dadurch gekennzeichnet, dass** die Durchbrechung (12) in dem undehnbaren Abschnitt (3g) des Gurtgrundkörpers (3a) ausgebildet ist.

## Claims

1. Orthopaedic bandage arrangement (1) for an ankle joint, comprising a knitted part (2) having a sock-like knitted main body (2a), and a strap part (3), having an elongate strap main body (3a), that is able to be joined to the knitted part (2), wherein at least one strap portion (3b) projects from the strap main body (3a), said strap portion (3b) extending over the outside of the foot in the worn state of the bandage arrangement (1), in which the strap part (3) is joined to the knitted part (2),
**characterized in that**
the strap portion (3b) extends over the ankle joint in the worn state of the bandage arrangement and is joined by its free end (3e) to the knitted main body (2a) and/or to the strap main body (3a) and as a result has a pronation effect on the ankle joint and provides supination protection for the ankle joint of the wearer.

2. Orthopaedic bandage arrangement according to Claim 1, **characterized in that** the strap portion (3b) projects from the strap main body (3a) at an angle (α) of between 10 and 90°, in particular between 20 and 45°, particularly preferably 25°.

3. Orthopaedic bandage arrangement according to Claim 1 or 2, **characterized in that** at least one fastening portion (7, 9), having a joining element, for releasably joining the strap part (3) to the knitted part (2) is provided in each case at the free ends (3c, 3d) of the strap main body (3a) and at the free end (3e) of the strap portion (3b), wherein preferably at least one fastening portion (7, 8) on the strap main-body side is joined releasably to the strap main body (3a) and/or preferably the fastening portion (9) on the strap-portion side is joined releasably to the strap portion (3b).

4. Orthopaedic bandage arrangement according to Claim 3, **characterized in that** the joining of the at least one fastening portion (8) to be joined to the strap main body (3a) and/or of the fastening portion (9) to be joined to the strap portion (3b) is formed via a joining element provided on the fastening-portion side and a joining element, corresponding thereto, on the strap main-body side and/or strap-portion side.

5. Orthopaedic bandage arrangement according to Claim 3 or 4, **characterized in that** the fastening portion (9) has a tongue (11) that projects therefrom and has a joining element, such that the fastening portion (9) is joinable to a corresponding joining element on the top side and underside of the strap main body (3a) and/or of the strap portion (3b).

6. Orthopaedic bandage arrangement according to one of the preceding claims, **characterized in that** a fastening portion (5) that is in particular wing-shaped and has a joining element is attached to the knitted main body (2a), said fastening portion (5) being joinable to a fastening portion (7) that is joined to a free end (3d) of the strap main body (3a) and has a corresponding joining element, wherein preferably the fastening portion (5) on the knitted main-body side is arranged on the outer periphery of the knitted main body (2a) and/or preferably the fastening portion (5) on the knitted main-body side is formed from an inelastic material.

7. Orthopaedic bandage arrangement according to one of the preceding claims, **characterized in that** the strap main body (3a) consists of an elastic portion (3f) and an inelastic portion (3g) joined thereto, wherein the strap portion (3b) projects from the inelastic portion (3g).

8. Orthopaedic bandage arrangement according to Claim 7, **characterized in that** the strap portion (3b) is formed integrally with the inelastic portion (3g), and/or the inelastic portion (3g) is provided with a joining element on its entire top side and underside.

9. Orthopaedic bandage arrangement according to one of Claims 3 to 8, **characterized in that** a joining element has a plurality of touch-and-close elements, in particular fleece elements or hook elements.

10. Orthopaedic bandage arrangement according to one of the preceding claims, **characterized in that** the strap main body (3a) has at least one elongate aperture (12) extending in particular in the longitudinal direction of the strap main body (3a).

11. Orthopaedic bandage arrangement according to one of Claims 7 to 9 and Claim 10, **characterized in that** the aperture (12) is formed in the inelastic portion (3g) of the strap main body (3a).

## Revendications

1. Agencement de bandage orthopédique (1) pour une cheville, comprenant une partie tricotée (2) présentant un corps de base tricoté de type chaussette (2a) et une partie de ceinture (3) présentant un corps de base de ceinture allongé (3a) et pouvant être assemblée à la partie tricotée (2), dans lequel au moins une partie de ceinture (3b) part du corps de base de ceinture (3a) et, dans l'état porté de l'agencement de bandage (1), dans lequel la partie de ceinture (3) est assemblée à la partie tricotée (2), s'étend sur le côté extérieur du pied, **caractérisé en ce que** la partie de ceinture (3b) dans l'état porté de l'agencement de bandage s'étend sur la cheville et est assemblée par son extrémité libre (3e) au corps de base tricoté (2a) et/ou au corps de base de ceinture (3a) et présente ainsi un effet de pronation sur la cheville et une protection contre la supination sur la cheville du porteur.

2. Agencement de bandage orthopédique selon la revendication 1, **caractérisé en ce que** la partie de ceinture (3b) part du corps de base de ceinture (3a) sous un angle (α) compris entre 10° et 90°, en particulier entre 20° et 45°, de préférence encore de 25°.

3. Agencement de bandage orthopédique selon la revendication 1 ou 2, **caractérisé en ce qu'**il est prévu aux extrémités libres (3c, 3d) du corps de base de ceinture (3a) ainsi qu'à l'extrémité libre (3e) de la partie de ceinture (3b) chaque fois au moins une partie de fixation (7, 9) présentant un élément d'assemblage pour l'assemblage détachable de la partie de ceinture (3) à la partie tricotée (2), dans lequel de préférence au moins une partie de fixation côté corps de base de ceinture (7, 8) est assemblée de façon détachable au corps de base de ceinture (3a) et/ou de préférence la partie de fixation côté partie de ceinture (9) est assemblée de façon détachable à la partie de ceinture (3b) .

4. Agencement de bandage orthopédique selon la revendication 3, **caractérisé en ce que** l'assemblage de ladite au moins une partie de fixation (8) à assembler au corps de base de ceinture (3a) et/ou de la partie de fixation (9) à assembler à la partie de ceinture (3b) est formé par un élément d'assemblage prévu côté partie de fixation et un élément d'assemblage correspondant du côté du corps de ceinture et/ou du côté de la partie de ceinture.

5. Agencement de bandage orthopédique selon la revendication 3 ou 4, **caractérisé en ce que** la partie de fixation (9) présente une languette (11) partant de celle-ci et présentant un élément d'assemblage, de telle manière que la partie de fixation (9) puisse être assemblée à un élément d'assemblage correspondant sur le côté supérieur et inférieur du corps de base de ceinture (3a) et/ou de la partie de ceinture (3b).

6. Agencement de bandage orthopédique selon l'une quelconque des revendications précédentes, **caractérisé en ce qu'**une partie de fixation (5), en particulier en forme d'aile, présentant un élément d'assemblage est appliquée sur le corps de base tricoté (2a), et peut être assemblée à une partie de fixation (7) assemblée à une extrémité libre (3d) du corps de base de ceinture (3a) et présentant un élément d'assemblage correspondant, dans lequel la partie de fixation côté corps de base tricoté (5) est disposée de préférence sur la périphérie extérieure du corps de base tricoté (2a), et/ou la partie de fixation côté corps de base tricoté (5) est formée d'un matériau non extensible.

7. Agencement de bandage orthopédique selon l'une quelconque des revendications précédentes, **caractérisé en ce que** le corps de base de ceinture (3a) se compose d'une partie extensible (3f) et d'une partie non extensible (3g) assemblée à celle-ci, dans lequel la partie de ceinture (3b) part de la partie non extensible (3g).

8. Agencement de bandage orthopédique selon la revendication 7, **caractérisé en ce que** la partie de ceinture (3b) est formée d'une pièce avec la partie non extensible (3g), et/ou la partie non extensible (3g) est munie complètement côté supérieur et inférieur d'un élément d'assemblage.

9. Agencement de bandage orthopédique selon l'une quelconque des revendications 3 à 8, **caractérisé en ce qu'**un élément d'assemblage présente plusieurs éléments de fermeture de type Velcro, en particulier des éléments en frise ou des éléments de crochets.

10. Agencement de bandage orthopédique selon l'une quelconque des revendications précédentes, **caractérisé en ce que** le corps de base de ceinture (3a) présente au moins une ouverture allongée (12), s'étendant en particulier dans la direction longitudinale du corps de base de ceinture (3a).

11. Agencement de bandage orthopédique selon l'une quelconque des revendications 7 à 9 et la revendication 10, **caractérisé en ce que** l'ouverture (12) est formée dans la partie non extensible (3g) du corps de base de ceinture (3a).
